# EUROPEAN PATENT APPLICATION

(11) **EP 4 417 127 A1**
(43) Date of publication of application: **21.08.2024**
(21) Application number: 22881248.3
(22) Date of filing: 23.09.2022
(51) Int. Cl.: A61B 5/346, A61B 5/349, A61B 5/02, G16H 50/20, G16H 50/70

(54) **METHOD, PROGRAM, AND DEVICE FOR DIAGNOSING LEFT VENTRICULAR SYSTOLIC DYSFUNCTION ON BASIS OF ELECTROCARDIOGRAM**

(30) Priority: 11.10.2021 KR 20210134573; 20.09.2022 KR 20220118528
(71) Applicant: Medical AI Co., Ltd., Seoul 06302 (KR)
(72) Inventor: KWON, Joonmyoung, Seoul 06302 (KR)
(74) Representative: Patentanwaltskanzlei Matschnig & Forsthuber OG
(86) International application number: PCT/KR2022/014267
(87) International publication number: WO 2023/063619

(57) **Abstract**

According to an embodiment of the present disclosure, there is provided a method of diagnosing left ventricular systolic dysfunction based on an electrocardiogram, the method being performed by a computing device including at least one processor, the method including: acquiring electrocardiogram data; and estimating the probability of occurrence of left ventricular systolic dysfunction for the subject of measurement of the electrocardiogram data based on the electrocardiogram data by using a pre-trained neural network model; wherein the neural network model has been trained based on the correlations between left ventricular systolic dysfunction and changes in electrocardiogram characteristics.

## Description

### Technical Field

The present disclosure relates to a method of diagnosing left ventricular systolic dysfunction, and more particularly to a method of diagnosing left ventricular systolic dysfunction based on an electrocardiogram by using a neural network model.

### Background Art

Electrocardiograms (ECGs) are signals used to determine the presence/absence of disease by checking for abnormalities in a conduction system from the heart to electrodes through the measurement of electrical signals generated in the heart.

The heartbeat, which is the cause of the generation of an electrocardiogram, is performed in such a manner that an impulse that originates from the sinus node located in the right atrium first depolarizes the right and left atria, and, after a brief delay in the atrioventricular node, activates the ventricles.

The right ventricle, which has the fastest septum and thin walls, activates before the left ventricle, which has thick walls. The depolarization waves transferred to the Purkinje fibers spread from the endocardium to the epicardium like wavefronts in the myocardium, thus causing ventricular contraction. Electrical impulses are normally conducted through the heart, and thus the heart contracts approximately 60 to 100 times per minute. Each contraction is represented by heart rate per beat.

Such electrocardiograms can be detected through bipolar leads, which record the potential differences between two portions, and unipolar leads, which record the potentials of the portions to which electrodes are attached. Methods of measuring electrocardiograms include standard limb leads, which are bipolar leads, unipolar limb leads, which are unipolar leads, and precordial leads, which are unipolar leads.

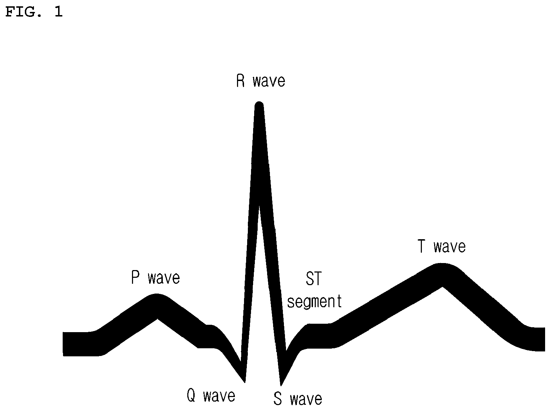

The electrical activity period of the heart is basically divided into atrial depolarization, ventricular depolarization, and ventricular repolarization stages. These individual stages are reflected in the shapes of several waves called P, Q, R, S, and T waves, as shown in FIG. 1.

The electrical activity of the heart can be considered to be normal only when these waves have standard shapes. In order to determine whether these waves have standard shapes, it is necessary to check whether characteristics, such as the times for which the individual waves are maintained, the intervals between the individual waves, the amplitudes of the individual waves, and kurtosis, are within normal ranges.

Such electrocardiogram is measured with an expensive measurement device and used as an auxiliary tool to measure the health condition of a patient. In general, the electrocardiogram measurement device only displays measurement results, and diagnosis is entirely the responsibility of a doctor.

Currently, research is continuing to rapidly and accurately diagnose diseases based on electrocardiograms by using artificial intelligence in order to reduce dependence on doctors. Furthermore, with the development of wearable, lifestyle electrocardiogram measurement devices, there is a rising possibility of diagnosing and monitoring not only heart diseases but also various other diseases based on electrocardiograms.

In particular, the severity of maternal death attributable to cardiovascular disease, represented by peripartum cardiomyopathy (PPCM), is emerging. According to existing criteria, peripartum cardiomyopathy is diagnosed as heart failure within the last month of pregnancy or within five months after delivery. Furthermore, the left ventricular ejection function (LVEF) of a patient is diagnosed when the LVEF is less than 45% by checking it through echocardiography. It is reported that peripartum cardiomyopathy has a particularly high incidence in Korea. Despite its severity, the awareness of peripartum cardiomyopathy is low, and diagnosis is difficult due to the similarities in signs and symptoms between normal pregnancy and heart failure.

### Disclosure

### Technical Problem

The present disclosure has been conceived in response to the above-described background technology, and an object of a method for diagnosing left ventricular systolic dysfunction based on an electrocardiogram according to an embodiment of the present disclosure is to provide a method for estimating the probability of occurrence of left ventricular systolic dysfunction for the subject of the measurement of electrocardiogram data based on the electrocardiogram data by using a neural network model.

However, the objects to be accomplished by the present disclosure are not limited to the object mentioned above, and other objects not mentioned may be clearly understood based on the following description.

### Technical Solution

According to an embodiment of the present disclosure for accomplishing the above-described object, there is provided a method of diagnosing left ventricular systolic dysfunction based on an electrocardiogram, the method being performed by a computing device including at least one processor, the method including: acquiring electrocardiogram data; and estimating the probability of occurrence of left ventricular systolic dysfunction for the subject of measurement of the electrocardiogram data based on the electrocardiogram data by using a pre-trained neural network model; wherein the neural network model has been trained based on the correlations between left ventricular systolic dysfunction and changes in electrocardiogram characteristics.

Alternatively, there may be provided the method, wherein the neural network model includes residual blocks each including a plurality of sub-modules and the neural network model receives the electrocardiogram data and outputs the probability of occurrence of peripartum cardiomyopathy.

Alternatively, there may be provided the method, wherein the sub-module includes a plurality of convolutional neural networks (CNNs), batch normalizations, and ReLU activation function layers, and further includes a dropout layer.

Alternatively, there may be provided the method, wherein the first sub-module of the sub-modules further includes a max pooling layer that directly performs input to the last one of the ReLU activation function layers.

Alternatively, there may be provided the method, wherein the neural network model further includes a fully connected layer into which auxiliary information is input and an output of the fully connected layer and outputs of the residual blocks are concatenated into one to derive the probability of occurrence of peripartum cardiomyopathy.

Alternatively, there may be provided the method, wherein the electrocardiogram data input to the neural network model is preprocessed by down-sampling and augmentation to which noise is applied and is then input to the residual blocks.

Alternatively, there may be provided the method, wherein the correlations between left ventricular systolic dysfunction and changes in electrocardiographic characteristics are based on electrocardiogram characteristics including at least one of the regions of PR segments, QRS segments, pathological Q waves, poor R progression, ST depression, T wave inversion, atrial premature beats, and ventricular premature beats.

According to another embodiment of the present disclosure, there is provided a computer program stored in a computer-readable storage medium, the computer program performing operations for diagnosing left ventricular systolic dysfunction based on an electrocardiogram when executed on one or more processors, wherein: the operations include operations of: acquiring electrocardiogram data; and estimating the probability of occurrence of left ventricular systolic dysfunction for the subject of measurement of the electrocardiogram data based on the electrocardiogram data by using a pre-trained neural network model; and the neural network model has been trained based on the correlations between left ventricular systolic dysfunction and changes in electrocardiogram characteristics.

According to another embodiment of the present disclosure, there is provided a computing device for diagnosing left ventricular systolic dysfunction based on an electrocardiogram, the computing device including: a processor including at least one core; and memory including program codes that are executable on the processor; wherein the processor, in response to the execution of the program codes, acquires electrocardiogram data, and estimates the probability of occurrence of left ventricular systolic dysfunction for the subject of measurement of the electrocardiogram data based on the electrocardiogram data by using a pre-trained neural network model; and wherein the neural network model has been trained based on the correlations between left ventricular systolic dysfunction and changes in electrocardiogram characteristics.

### Advantageous Effects

The method for diagnosing left ventricular systolic dysfunction based on an electrocardiogram according to an embodiment of the present disclosure has the effect of providing the method for estimating the probability of occurrence of left ventricular systolic dysfunction for the subject of the measurement of electrocardiogram data based on the electrocardiogram data by using the neural network model.

### Description of Drawings

FIG. 1 is a diagram showing an electrocardiogram signal according to the present disclosure;
FIG. 2 is a block diagram of a computing device according to an embodiment of the present disclosure;
FIG. 3 is a flowchart showing a method of diagnosing left ventricular systolic dysfunction based on an electrocardiogram according to an embodiment of the present disclosure;
FIG. 4 is a diagram showing the architecture of a neural network model according to an embodiment of the present disclosure;
FIG. 5 is a diagram showing a process for internal validation tests of a neural network model according to an embodiment of the present disclosure;
FIG. 6 is a diagram showing a process for external validation tests of a neural network model according to an embodiment of the present disclosure;
FIG. 7 is a diagram showing the results of internal verification tests of a neural network model according to an embodiment of the present disclosure;
FIG. 8 is a diagram showing the results of external verification tests of a neural network model according to an embodiment of the present disclosure; and
FIGS. 9 and 10 are diagrams showing the visualization of the training results of a neural network model according to an embodiment of the present disclosure.

### Mode for Invention

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "n-th (n is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "acquisition" used herein can be understood to mean not only receiving data over a wired/wireless communication network connecting with an external device or a system, but also generating data in an on-device form.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

"Data" used herein may include "images," signals, etc. The term "image" used herein may refer to multidimensional data composed of discrete image elements. In other words, "image" may be understood as a term referring to a digital representation of an object that can be seen by the human eye. For example, "image" may refer to multidimensional data composed of elements corresponding to pixels in a two-dimensional image. "Image" may refer to multidimensional data composed of elements corresponding to voxels in a three-dimensional image.

The term "block" used herein may be understood as a set of components classified based on various criteria such as type, function, etc. Accordingly, the components classified as each "block" may be changed in various manners depending on the criteria. For example, a neural network "block" may be understood as a set of neural networks including one or more neural networks. In this case, it can be assumed that the neural networks included in the neural network "block" perform the same specific operations. The foregoing descriptions of the terms are intended to help to understand the present disclosure. Accordingly, it should be noted that unless the above-described terms are explicitly described as limiting the content of the present disclosure, the terms in the content of the present disclosure are not used in the sense of limiting the technical spirit of the present disclosure.

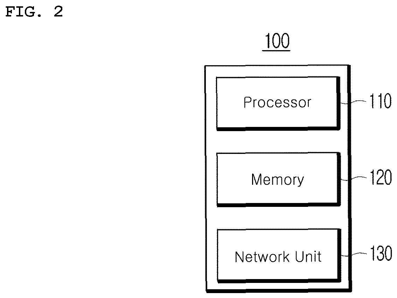

FIG. 2 is a block diagram of a computing device according to an embodiment of the present disclosure.

A computing device 100 according to an embodiment of the present disclosure may be a hardware device or part of a hardware device that performs the comprehensive processing and calculation of data, or may be a software-based computing environment that is connected to a communication network. For example, the computing device 100 may be a server that performs an intensive data processing function and shares resources, or may be a client that shares resources through interaction with a server. Furthermore, the computing device 100 may be a cloud system in which a plurality of servers and clients interact with each other and comprehensively process data. Since the above descriptions are only examples related to the type of computing device 100, the type of computing device 100 may be configured in various manners within a range understandable to those skilled in the art based on the content of the present disclosure.

Referring to FIG. 2, the computing device 100 according to an embodiment of the present disclosure may include a processor 110, memory 120, and a network unit 130. However, FIG. 1 shows only an example, and the computing device 100 may include other components for implementing a computing environment. Furthermore, only some of the components disclosed above may be included in the computing device 100.

The processor 110 according to an embodiment of the present disclosure may be understood as a configuration unit including hardware and/or software for performing computing operation. For example, the processor 110 may read a computer program and perform data processing for machine learning. The processor 110 may process computational processes such as the processing of input data for machine learning, the extraction of features for machine learning, and the calculation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure.

The processor 110 may train a neural network model that diagnoses left ventricular systolic dysfunction based on medical data. For example, the processor 110 may train the neural network model to estimate the occurrence and progress of left ventricular systolic dysfunction based on biological data including information such as gender, age, weight, height, etc., together with electrocardiogram data. More specifically, the processor 110 may train the neural network model so that the neural network model detects changes in electrocardiograms attributable to left ventricular systolic dysfunction by inputting electrocardiogram data and various types of biological data to the neural network model. In this case, the neural network model may be trained based on the correlations between left ventricular systolic dysfunction and changes in electrocardiograms. The correlations between left ventricular systolic dysfunction and changes in electrocardiograms may be understood as information about the relationships between changes in cardiac function and morphological changes in electrocardiogram signals. The processor 110 may perform an operation representative of at least one neural network block included in the neural network model during the process of training the neural network model.

The processor 110 may estimate whether left ventricular systolic dysfunction has occurred based on medical data by using the neural network model generated through the above-described training process. The processor 110 may generate inference data representative of the results of estimation of the probability of occurrence of left ventricular systolic dysfunction in a person by inputting electrocardiogram data and biological data including information such as gender, age, weight, height, etc. to the neural network model trained through the above-described process. For example, the processor 110 may predict the occurrence and progress of left ventricular systolic dysfunction by inputting electrocardiogram data to the trained neural network model. The processor 110 may accurately predict the occurrence of left ventricular systolic dysfunction by effectively identifying subtle changes in electrocardiograms that are difficult for humans to interpret through the neural network model for diagnosing left ventricular systolic dysfunction.

In addition to the examples described above, the types of medical data and the output of the neural network model may be configured in various manners within a range understandable to those skilled in the art based on the content of the present disclosure.

The memory 120 according to an embodiment of the present disclosure may be understood as a configuration unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize, and manage data necessary for the processor 110 to perform computation, the combination of data, and program codes executable on the processor 110. For example, the memory 120 may store medical data received through the network unit 130, which will be described later. The memory 120 may store program codes configured to operate the neural network model to receive medical data and perform learning, program codes configured to operate the neural network model to receive medical data and perform inference in accordance with the purpose of use of the computing device 100, processed data generated as program codes are executed, etc.

The network unit 130 according to an embodiment of the present disclosure may be understood as a configuration unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, a ultra wide-band wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive data necessary for the processor 110 to perform computation through wired/wireless communication with any system or client or the like. Furthermore, the network unit 130 may transmit data generated through the computation of the processor 110 through wired/wireless communication with any system or client or the like. For example, the network unit 130 may receive medical data through communication with a database within a hospital environment, a cloud server configured to perform tasks such as the standardization of medical data, a computing device, or the like. The network unit 130 may transmit the output data of the neural network model, intermediate data and processed data acquired from the computation process of the processor 110, etc. through communication with the above-described database, server, or computing device.

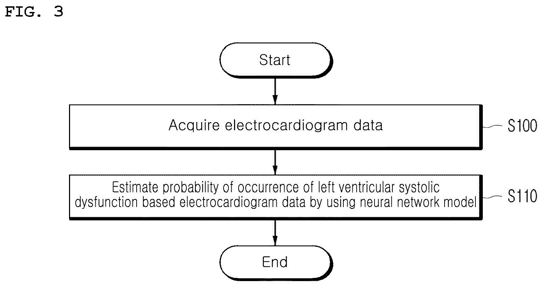

FIG. 3 is a flowchart showing a method of diagnosing thyroid dysfunction based on an electrocardiogram according to an embodiment of the present disclosure.

Referring to FIG. 3, there is shown a method of diagnosing left ventricular systolic dysfunction based on an electrocardiogram, which is performed by a computing device including at least one processor. First, there may be performed step S100 of acquiring electrocardiogram data.

The electrocardiogram data may be acquired directly as the data measured through an electrocardiogram measurement device, or may be acquired from an electrocardiogram measurement device through network communication.

Next, there may be performed step S110 of estimating the probability of occurrence of left ventricular systolic dysfunction for the subject of measurement of the electrocardiogram data based on the electrocardiogram data by using a pre-trained neural network model.

Furthermore, the estimation step S110 may include the step of estimating the probability of occurrence of left ventricular systolic dysfunction for the subject of measurement of the electrocardiogram data by inputting biological data including at least one of age, gender, weight, and height, together with the electrocardiogram data, to the neural network model.

In this case, the neural network model may be trained based on the correlations between left ventricular systolic dysfunction and changes in characteristics. Furthermore, the neural network model may be trained based on the correlations between left ventricular systolic dysfunction and changes in characteristics such as electrocardiograms, gender, age, weight, and height. More specifically, the neural network model may be trained based on the correlations between the occurrence and progress of left ventricular systolic dysfunction during a delivery week and changes in electrocardiograms or other characteristics. The neural network model may be used to diagnose not only peripartum left ventricular systolic dysfunction, but also various types of left ventricle-related dysfunction.

The neural network model may be trained based on electrocardiograms measured with 12 leads and acquired from electrodes of an electrocardiogram measurement device connected to the human body. For example, an electrocardiogram may be measured with 12 leads for ten seconds in length, and may be stored as 500 points per second. Additionally, the neural network model may be trained based on partial information acquired by extracting only 6-limb lead electrocardiograms and single-lead (lead I) electrocardiograms from 12-lead electrocardiograms.

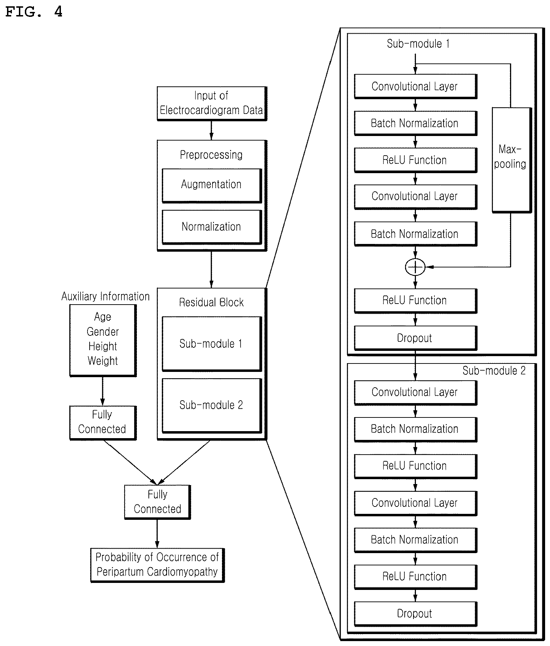

Referring to FIG. 4, there is shown a diagram showing the architecture of a neural network model according to an embodiment of the present disclosure.

Referring to FIG. 4, the neural network model according to the embodiment of the present disclosure may include a neural network composed of a plurality of residual blocks. The neural network composed of residual blocks may be intended to receive electrocardiogram data and output the probability of occurrence of peripartum left ventricular systolic dysfunction.

More specifically, the neural network model may include residual blocks each including a plurality of sub-modules. The neural network model may receive electrocardiogram data and output the probability of occurrence of peripartum cardiomyopathy. The sub-modules each include a plurality of convolutional neural networks (CNNs), batch normalizations, and ReLU activation function layers, and may further include a dropout layer.

A first sub-module may include a max pooling layer that directly performs input to a last ReLU activation function layer. The neural network model may include a fully connected layer to which auxiliary information, such as age, gender, height, weight, etc., is input. The output of the fully connected layer and the outputs of the residual blocks may be concatenated into one to derive the probability of occurrence of peripartum cardiomyopathy.

The electrocardiogram data input to the neural network model may be preprocessed by down-sampling and augmentation to which noise is applied and then be input to the residual blocks. More specifically, the sampling rate of the electrocardiogram is down-sampled from 500 Hz to 250 Hz and data augmentation is used with various types of noise applied thereto.

The neural network model may include neural networks corresponding to the plurality of leads of electrocardiogram data, respectively. That is, the neural network model may include individual neural networks to which electrocardiograms measured with individual leads are input.

For example, the neural network model may include a first sub-neural network model that has been trained based on electrocardiogram data measured with 12 multiple leads. Furthermore, the neural network model may further include a second sub-neural network model that has been trained based on at least six limb leads or six precordial leads. Furthermore, the neural network model may further include a third sub-neural network model that has been trained based on electrocardiogram data measured with single leads. The neural network model may selectively use at least one of the first, second, and third sub-neural network models depending on the number of leads. Accordingly, the neural network model may effectively predict the occurrence of left ventricular systolic dysfunction regardless of the number of leads. Furthermore, when 12-lead electrocardiogram data is input, the neural network model may output the probability of occurrence of left ventricular systolic dysfunction by combining the outputs of the respective sub-models using all of the first, second, and third sub-neural network models. The neural network model may increase the accuracy of prediction of the occurrence of left ventricular systolic dysfunction through this combination.

A statistical analysis method performed to validate a neural network model having the above-described architecture will be described below.

The performance of the neural network model was measured in terms of sensitivity, specificity, a positive predictive value (PPV), and a negative predictive value (NPV) with 95% confidence intervals (95% CI) for first and second test results. Area under the receiver operating characteristic curve (AUROC) calculation and receiver operating characteristic curve (AUC) analysis were also performed with 95% CI by using Youden's J statistic. Variables were compared using a student's t-test, a Mann-Whitney U test, a chi-square test (a χ2 test), and a Fisher's exact test as appropriate. The P-value was set to a value less than 0.05.

A validation study of a neural network model according to an embodiment of the present disclosure will be described below.

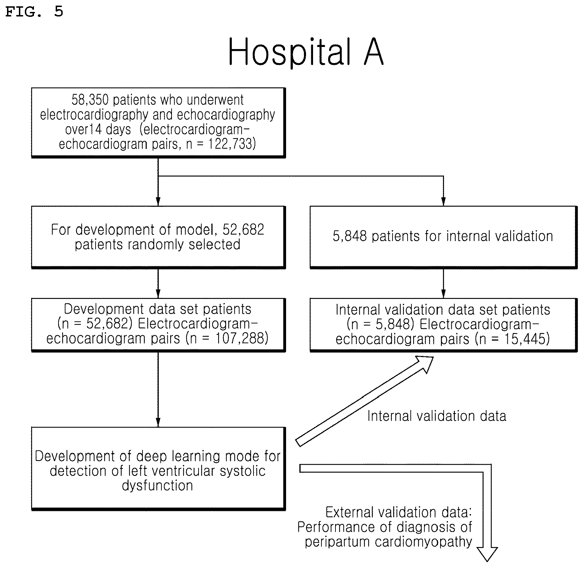

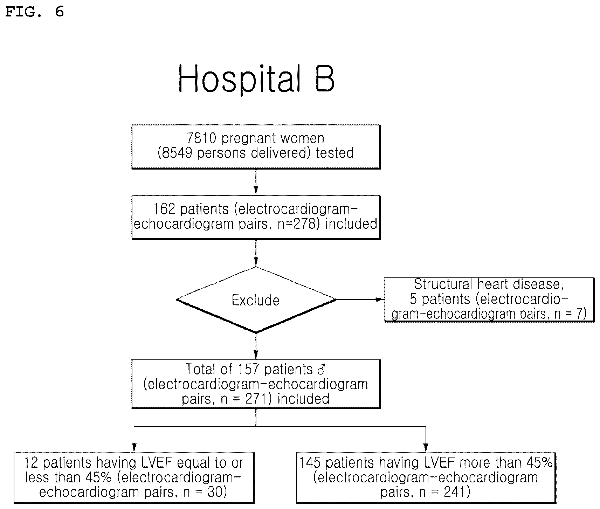

FIG. 5 is a diagram showing a process for internal validation tests of a neural network model according to an embodiment of the present disclosure. FIG. 6 is a diagram showing a process for external validation tests of a neural network model according to an embodiment of the present disclosure.

Referring to FIGS. 5 and 6, 278 electrocardiogram-echocardiogram pairs out of 8,549 delivery cases were used as data for validation study. Seven couples (five pregnant women) having pre-existing structural heart disease, congenital heart disease, or newly confirmed heart disease on perinatal echocardiography were excluded. A total of 271 couples (157 pregnant women) were included as a main study population.

Referring to Table 1 below, the average age of the study target patients was 33.4 years old, and 50 patients (31.8%) were elderly (> 35 years old). Excluding 45 patients having no recorded obstetric history, the primiparous and multiparous proportion were 48.2% and 51.8%, respectively.

**Table 1**

| | Excluded | Total (n=157) | LVEF 45% or less (n=12) | LVEF > 45% (n=145) | P-value |
|---|---|---|---|---|---|
| Demographics | | | | | |
| Age, mean (SD) | 0 | 33.4 (4.3) | 32.9 (5.0) | 33.5 (4.3) | 0.715 |
| Old age (>35 years), n (%) | 0 | 50 (31.8) | 3 (25.0) | 47 (32.4) | 0.753 |
| BMI, kg/m^2, mean (SD) | 0 | 27.59 (4.5) | 27.6 (4.4) | 28.1 (5.2) | 0.730 |

| Medical history | | | | | |
|---|---|---|---|---|---|
| Primiparity, n (%) | 45 | 54 | 6 (66.7) | 48 (46.6) | 0.309 |
| Multiparity, n (%) | | 58 | 3 (33.3) | 55 (53.4) | |
| Cesarean section, n (%) | 0 | 14 (8.9) | 2 (16.7) | 12 (8.3) | 0.291 |
| Normal delivery, n (%) | 0 | 143 (91.1) | 10 (83.3) | 133 (91.7) | |
| Preterm labor, n (%) | 0 | 28 (17.8) | 3 (25.0) | 25 (17.2) | 0.450 |
| Diabetes, n (%) | 0 | 10 (6.4) | 2 (16.7) | 8 (5.5) | 0.171 |
| Chronic hypertension, n (%) | 0 | 15 (9.6) | 1 (8.3) | 14 (9.7) | 1.000 |
| Gestational hypertension, n (%) | 0 | 44 (28.0) | 4 (33.3) | 40 (27.6) | 0.740 |
| Preeclampsia, n (%) | 0 | 33 (21.0) | 4 (33.3) | 29 (20.0) | 0.279 |
| Eclampsia, n (%) | 0 | 1 (0.6) | 1 (8.3) | 0 (0.0) | 0.076 |

| Experimental Finding | | | | | |
|---|---|---|---|---|---|
| BNP, pg/mL, mean (SD) | 146 | 839.1 (1145.6) | 1547.0 (1438.2) | 249.2 (227.0) | 0.114 |
| proBNP, pg/mL, mean (SD) | 139 | 3261.8 (5075.3) | 3135.2 (940.9) | 3297.9 (5785.7) | 0.921 |
| pre-Hb, g/dL, mean (SD) | 59 | 11.4 (2.1) | 11.6 (3.1) | 11.3 (1.9) | 0.830 |
| post-Hb, g/dL, mean (SD) | 2 | 9.9 (1.9) | 9.2 (2.4) | 9.9 (1.9) | 0.362 |

| Medicine | | | | | |
|---|---|---|---|---|---|
| Magnesium sulfate, n (%) | 0 | 53 (33.8) | 6 (50.0) | 47 (32.4) | 0.222 |
| Labetalol, n (%) | 0 | 11 (7.0) | 1 (8.3) | 10 (6.9) | 0.595 |
| Hydralazine, n (%) | 0 | 39 (24.8) | 5 (41.7) | 34 (23.4) | 0.174 |
| Nifedipine, n (%) | 0 | 31 (19.7) | 5 (41.7) | 26 (17.9) | 0.061 |
| Aspirin, n (%) | 0 | 5 (3.2) | 1 (8.3) | 4 (2.8) | 0.332 |
| Ritodrine, n (%) | 0 | 19 (12.1) | 0 (0.0) | 19 (13.1) | 0.363 |
| Atosiban, n (%) | 0 | 4 (2.5) | 0 (0.0) | 4 (2.8) | 1.000 |
| % values exclude missing values. | | | | | |
| LVEF = left ventricular ejection fraction, | | | | | |
| BMI = body mass index, | | | | | |
| BNP = b-type natriuretic peptide, | | | | | |
| Pre-Hb = hemoglobin level before delivery or surgery, | | | | | |
| Post-Hb = hemoglobin level after delivery or surgery. | | | | | |

The neural network model was trained based on a dataset of 107,288 electrocardiograms for 52,682 patients. Internal validation tests were conducted using 15,445 pieces of electrocardiogram data from 5,848 patients. The basic characteristics of the cohort used for the training of the neural network model can be found in Table 2 below:

**Table 2**

| | LVEF > 45% (n=88,449) | LVEF 45% or less (n=18,839) | P-value |
|---|---|---|---|
| Age, mean (SD) | 60.5 ± 15.0 | 65.0 ± 13.6 | <0.001 |
| Female | 44,721 (50.6%) | 11,992 (63.7%) | <0.001 |
| BMI, kg/m^2, mean (SD) | 24.6 ± 3.7 | 24.2 ± 3.9 | <0.001 |
| Time difference, day (SD) | 0.7 ± 5.7 | -1.3 ± 5.2 | <0.001 |
| LVEF, % (SD) | 59.6 ± 7.0 | 33.4 ± 9.5 | <0.001 |
| Left atrial dimension, mm (SD) | 39.8 ± 8.3 | 45.7 ± 9.6 | <0.001 |
| Septal thickness, mm (SD) | 10.1 ± 1.9 | 10.3 ± 1.8 | <0.001 |
| Posterior wall thickness, mm (SD) | 9.6 ± 1.5 | 10.0 ± 2.1 | <0.001 |
| Aortic dimension, mm (SD) | 31.6 ± 4.2 | 32.9 ± 4.5 | <0.001 |
| E speed, mm/sec (SD) | 65.7 ± 21.4 | 70.7 ± 27.9 | <0.001 |
| A speed, mm/sec (SD) | 71.8 ± 21.5 | 69.6 ± 24.8 | <0.001 |
| Deceleration time, ms (SD) | 207.5 ± 59.0 | 183.3 ± 66.0 | <0.001 |
| E' speed, cm/sec (SD) | 6.7 ± 2.5 | 5.0 ± 1.9 | <0.001 |
| A' speed, cm/sec (SD) | 8.7 ± 2.1 | 6.9 ± 2.4 | <0.001 |
| Peak TRPG, mmHg (SD) | 21.6 ± 7.6 | 26.4 ± 11.7 | <0.001 |
| Expected PA pressure, mmHg (SD) | 25.2 ± 8.6 | 31.8 ± 13.7 | <0.001 |
| Left ventricular systolic dimension, mm (SD) | 29.5 ± 5.9 | 43.0 ± 11.4 | <0.001 |
| Left ventricular diastolic dimension, mm (SD) | 47.3 ± 5.1 | 55.5 ± 8.8 | <0.001 |
| Heart rate, beat/min (SD) | 73.1 ± 16.8 | 81.8 ± 21.6 | <0.001 |
| QTc interval, ms (SD) | 439.5 ± 35.4 | 473.8 ± 44.1 | <0.001 |
| P axis, degree (SD) | 44.1 ± 30.8 | 43.8 ± 39.3 | 0.325 |
| QRS axis, degree (SD) | 38.0 ± 43.9 | 28.0 ± 65.3 | <0.001 |
| T axis, degree (SD) | 46.3 ± 52.5 | 82.1 ± 84.4 | <0.001 |
| DLM = neural network model | | | |
| LVEF = left ventricular ejection fraction | | | |
| BMI = body mass index | | | |
| Time difference = echocardiogram time - electrocardiogram time | | | |
| TRPG = tricuspid regurgitation pressure gradient | | | |
| PA = pulmonary artery | | | |

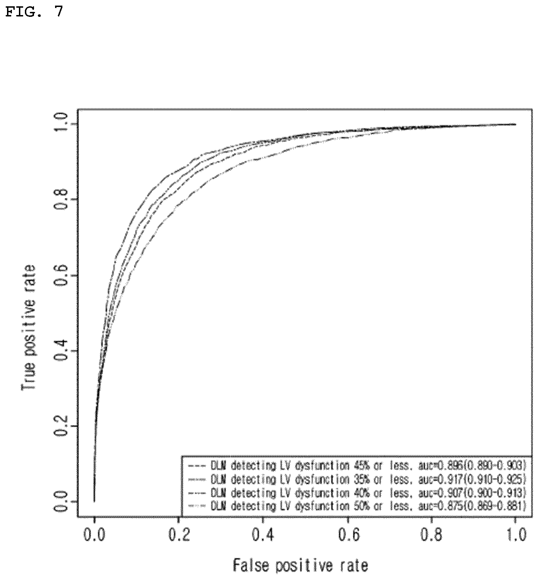

FIG. 7 is a diagram showing the results of internal verification tests of a neural network model according to an embodiment of the present disclosure. Referring to FIG. 7, the area under the receiver operating characteristic curve (hereinafter referred to as AUROC), sensitivity, specificity, PPV, and NPV of the neural network model for the detection of heart failure during internal validation tests can be seen in Table 3 below. It can be seen that for LVEF ≤ 45%, AUROC was 0.896 (95% confidence interval [CI], 0.890-0.903), sensitivity was 0.796 (95% CI, 0.781-0.811), specificity was 0.841 (95% CI, 0.8435), PPV was 0.533 (95% CI, 0.518-0.548), and NPV was 0.948 (95% CI, 0.944-0.952).

**Table 3**

| | AUROC (95% CIs) | Sensitivity (95% CIs) | Specificity (95% CIs) | PPV (95% CIs) | NPV (95% CIs) |
|---|---|---|---|---|---|
| LVEF 35% or less | 0.917 (0.910-0.925) | 0.852 (0.835-0.870) | 0.840 (0.834-0.846) | 0.369 (0.353-0.385) | 0.981 (0.979-0.983) |
| LVEF 40% or less | 0.907 (0.900-0.913) | 0.860 (0.845-0.874) | 0.796 (0.789-0.803) | 0.398 (0.384-0.412) | 0.973 (0.970-0.976) |
| LVEF 45% or less | 0.896 (0.890-0.903) | 0.796 (0.781-0.811) | 0.841 (0.835-0.848) | 0.533 (0.518-0.548) | 0.948 (0.944-0.952) |
| LVEF 50% or less | 0.875 (0.869-0.881) | 0.784 (0.771-0.797) | 0.803 (0.795-0.810) | 0.583 (0.570-0.596) | 0.914 (0.908-0.919) |

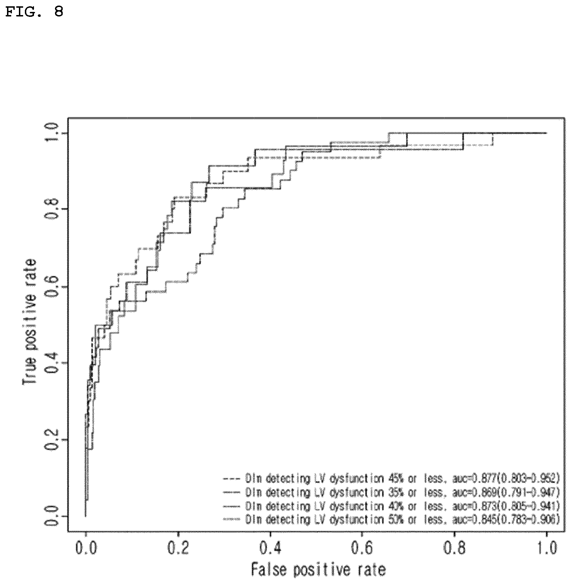

FIG. 8 is a diagram showing the results of external verification tests of a neural network model according to an embodiment of the present disclosure.

Referring to FIG. 8, using the cutoff value of the peripartum cardiomyopathy criteria, the AUROC of the neural network model for detecting peripartum cardiomyopathy at LVEF of 45% or less was 0.877 (95% CI, 0.803-0.952). Furthermore, referring to Table 4 below, sensitivity, specificity, PPV, and NPV were measured as 0.833 (95% CI, 0.700-0.967), 0.809 (95% CI, 0.760-0.859), 0.352 (95% CI, 0.2491-0.2491-0.453), and 0.975 (95% CI, 0.953-0.997), respectively.

**Table 4**

| | | AUROC (95% CIs) | Sensitivity (95% CIs) | Specificity (95% CIs) | PPV (95% CIs) | NPV (95% CIs) |
|---|---|---|---|---|---|---|
| Primary outcome | LVEF 45% or less | 0.877 (0.803-0.952) | 0.833 (0.700-0.967) | 0.809 (0.760-0.859) | 0.352 (0.241-0.463) | 0.975 (0.953-0.997) |
| Secondary outcome | LVEF 35% or less | 0.869 (0.791-0.947) | 0.913 (0.798-1.028) | 0.734 (0.679-0.789) | 0.241 (0.151-0.331) | 0.989 (0.974-1.004) |
| | LVEF 40% or less | 0.873 (0.805-0.941) | 0.821 (0.680-0.963) | 0.815 (0.766-0.864) | 0.338 (0.226-0.451) | 0.975 (0.954-0.997) |
| | LVEF 50% or less | 0.845 (0.783-0.906) | 0.854 (0.745-0.962) | 0.657 (0.595-0.718) | 0.307 (0.222-0.392) | 0.962 (0.932-0.992) |

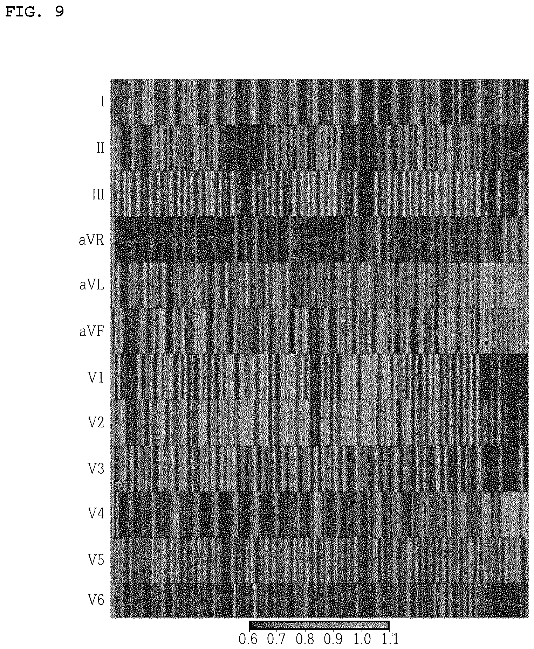

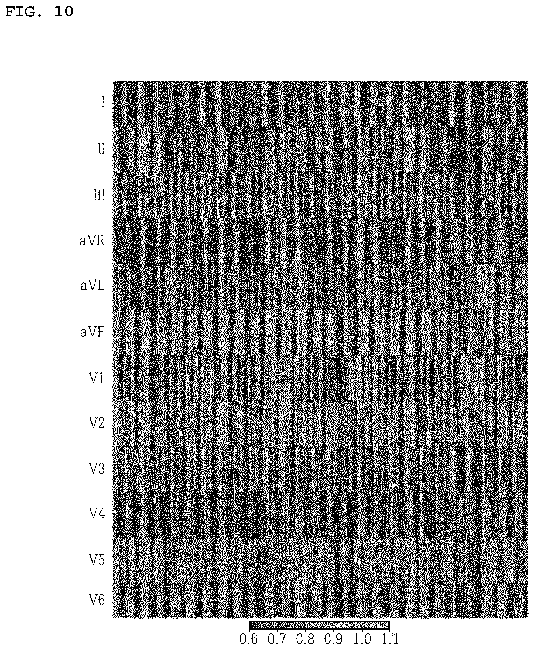

FIGS. 9 and 10 are diagrams showing the visualization of the training results of a neural network model according to an embodiment of the present disclosure.

Referring to FIGS. 9 and 10, it can be seen that gradient-weighted CAM (Grad-CAM) was applied to identify the electrocardiogram region on which the neural network model focused. It is determined that the neural network model focused on the regions of PR segments, QRS segments, pathological Q waves, poor R wave progression, ST depression, T wave inversion, atrial premature beats, and ventricular premature beats in order to determine the probability of occurrence of peripartum cardiomyopathy.

In summary, the above-described correlations between left ventricular systolic dysfunction and changes in electrocardiographic characteristics may be based on electrocardiogram characteristics including at least one of the regions of PR segments, QRS segments, pathological Q waves, poor R progression, ST depression, T wave inversion, atrial premature beats, and ventricular premature beats.

As described above, according to the method of diagnosing left ventricular systolic dysfunction according to the present disclosure, the neural network model was developed to predict the probability of occurrence of peripartum cardiomyopathy, and the neural network model was externally validated using 271 electrocardiogram-echocardiography pairs.

During pregnancy, hemodynamic and structural changes in the body may occur. Hormonal changes, together with structural changes in the left ventricle, may contribute to a decrease in systemic vascular resistance as well as increases in plasma volume and cardiac output.

A study was conducted to demonstrate the suitability of the neural network model based on electrocardiograms for pregnant patients compared to general patients, with the characteristics of pregnancy being taken into consideration. It was found that there were changes in electrocardiograms during pregnancy, and also it was determined that an increase in the heart rate, a change in the QRS axis, a change in the non-specific ST segment, and a change in the T wave axis occurred.

The method of diagnosing left ventricular systolic dysfunction based on an electrocardiogram according to an embodiment of the present disclosure may estimate the probability of occurrence of left ventricular systolic dysfunction for the subject of the measurement of electrocardiogram data based on the electrocardiogram data by using the neural network model.

Furthermore, the method for diagnosing left ventricular systolic dysfunction according to an embodiment of the present disclosure has the effect of diagnosing left ventricular systolic dysfunction based on information such as electrocardiograms, gender, age, height, weight, etc. by using the neural network model.

The various embodiments of the present disclosure described above may be combined with one or more additional embodiments, and may be changed within the range understandable to those skilled in the art in light of the above detailed description. The embodiments of the present disclosure should be understood as illustrative but not restrictive in all respects. For example, individual components described as unitary may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

## Claims

1. A method of diagnosing left ventricular systolic dysfunction based on an electrocardiogram, the method being performed by a computing device including at least one processor, the method comprising:
acquiring electrocardiogram data; and
estimating a probability of occurrence of left ventricular systolic dysfunction for a subject of measurement of the electrocardiogram data based on the electrocardiogram data by using a pre-trained neural network model;
wherein the neural network model has been trained based on correlations between left ventricular systolic dysfunction and changes in electrocardiogram characteristics.

2. The method of claim 1, wherein:
the neural network model includes residual blocks each including a plurality of sub-modules; and
the neural network model receives the electrocardiogram data and outputs a probability of occurrence of peripartum cardiomyopathy.

3. The method of claim 2, wherein the sub-module includes a plurality of convolutional neural networks (CNNs), batch normalizations, and ReLU activation function layers, and further includes a dropout layer.

4. The method of claim 3, wherein a first sub-module of the sub-modules further includes a max pooling layer that directly performs input to a last one of the ReLU activation function layers.

5. The method of claim 2, wherein:
the neural network model further includes a fully connected layer into which auxiliary information is input; and
an output of the fully connected layer and outputs of the residual blocks are concatenated into one to derive the probability of occurrence of peripartum cardiomyopathy.

6. The method of claim 2, wherein the electrocardiogram data input to the neural network model is preprocessed by down-sampling and augmentation to which noise is applied and is then input to the residual blocks.

7. The method of claim 1, wherein the correlations between left ventricular systolic dysfunction and changes in electrocardiographic characteristics are based on electrocardiogram characteristics including at least one of regions of PR segments, QRS segments, pathological Q waves, poor R progression, ST depression, T wave inversion, atrial premature beats, and ventricular premature beats.

8. A computer program stored in a computer-readable storage medium, the computer program performing operations for diagnosing left ventricular systolic dysfunction based on an electrocardiogram when executed on one or more processors, wherein:
the operations include operations of:
acquiring electrocardiogram data; and
estimating a probability of occurrence of left ventricular systolic dysfunction for a subject of measurement of the electrocardiogram data based on the electrocardiogram data by using a pre-trained neural network model; and
the neural network model has been trained based on correlations between left ventricular systolic dysfunction and changes in electrocardiogram characteristics.

9. A computing device for diagnosing left ventricular systolic dysfunction based on an electrocardiogram, the computing device comprising:
a processor including at least one core; and
memory including program codes that are executable on the processor;
wherein the processor, in response to execution of the program codes, acquires electrocardiogram data, and estimates a probability of occurrence of left ventricular systolic dysfunction for a subject of measurement of the electrocardiogram data based on the electrocardiogram data by using a pre-trained neural network model; and
wherein the neural network model has been trained based on correlations between left ventricular systolic dysfunction and changes in electrocardiogram characteristics.
